# EUROPÄISCHE PATENTSCHRIFT

(11) **EP 0 381 622 B1**
(45) Veröffentlichungstag und Bekanntmachung des Hinweises auf die Patenterteilung: **16.03.1994**
(21) Anmeldenummer: 90810053.0
(22) Anmeldetag: 23.01.1990
(51) Int. Cl.: C07C 47/14, C07C 45/65

(54) **Verfahren zur Herstellung von Dihalogenbutyraldehyden**
Method for the production of dihalogenobutyraldehydes
Procédé pour la fabrication de dihalogénobutyraldéhydes

(30) Priorität: 02.02.1989 CH 364/89
(43) Veröffentlichungstag der Anmeldung: 08.08.1990
(73) Patentinhaber: CIBA-GEIGY AG, 4002 Basel (CH)
(72) Erfinder: Steiner, Heinz, CH-4142 Münchenstein (CH); Tobler, Hans, Dr., CH-4123 Allschwil (CH)

(56) Entgegenhaltungen:
- DE-A- 2 062 915
- DE-A- 2 446 587
- US-A- 4 691 052
- TEILHEIMER: "SYNTHETIC METHODS OF ORGANIC CHEMISTRY, Band 39, Seite 38, 1985, Verlag S. Karger, Basel, CH
- HOUBEN-WEYL: "METHODEN DER ORGANISCHEN CHEMIE", 4. Auflage, Band IV/1c, 1980, Seiten 368-373, Georg Thieme Verlag, Stuttgart, DE
- SYNTHESIS, Nr. 12, Dezember 1985, Seite 1120, Stuttgart, DE; J.C. BLAZEJEWSKI: "Synthesis of 2-Trifluoromethyl-1,3-cyclopentanedione and 2-Trifluoromethyl-1,4-benzoquinone via Chlorous Acid Oxidation of 3-Trifluoromethylphenol"

## Beschreibung

Die Erfindung betrifft ein Verfahren zur Herstellung von in 2,4-Stellung chlorierten und/oder bromierten Butyraldehyden durch katalytische Dehalogenierung mit Wasserstoff in Gegenwart eines Halogenwasserstofffängers, bei dem man einen entsprechenden Butyraldehyd mit insgesamt zwei in 2-Stellung gebundenen Cl- und/oder Br-Atomen in einem organischen aprotischen Lösungsmittel umsetzt.

In Houben-Weyl, Bd. 4/1C, Seite 373 (1980) ist die Umsetzung von 2,2-Dichlor-ε-caprolactam mit Wasserstoff in Gegenwart von Pd/C und Natriumacetat und in Eisessig als Lösungsmittel zu 2-Chlor-ε-caprolactam beschrieben. M. Brenner et al. beschreiben in Helv. Chem. Acta, 21, S. 185 (1958) die gleiche Reaktion mit Raney-Nickel, Triethanolamin und Methanol als Lösungsmittel. Die Umsetzungen liefern das gewünschte 2-Chlor-ε-caprolactam in mässigen bis guten Umsätzen bei mässigen Selektivitäten. Die katalytische Umsetzung von α,α-Dihalogenaldehyden mit Wasserstoff ist noch nicht beschrieben worden.

Es wurde gefunden, dass α,α-dihalogenierte Butyraldehyde bei hohen chemischen Umsätzen und hohen Selektivitäten katalytisch mit Wasserstoff in Gegenwart eines Halogenwasserstoffängers zu den entsprechenden α-Monohalogenbutyraldehyden umgesetzt werden können, wenn man als Lösungsmittel organische aprotische Lösungsmittel verwendet.

Gegenstand der Erfindung ist ein Verfahren zur Herstellung von Dihalogenbutyraldehyden der Formel I 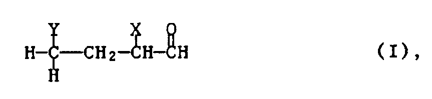
worin
X und Y unabhängig voneinander -Cl oder -Br darstellen,
durch katalytische Dehalogenierung von α,α-Dihalogenverbindungen mit Wasserstoff in Gegenwart eines Edelmetallkatalysators oder Raney-Nickel, eines Halogenwasserstoffängers und eines Lösungsmittels bei einer Temperatur von 0-150°C und Normaldruck oder einem Druck bis zu 15 MPa, das dadurch gekennzeichnet ist, dass man als α,α-Dihalogenverbindung einen α,α-Dihalogenbutyraldehyd der Formel II 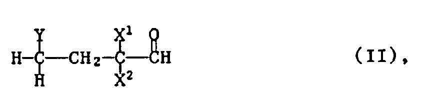
worin Y die zuvor angegebene Bedeutung hat und X¹ und X² unabhängig voneinander -Cl oder -Br darstellen, verwendet und die Reaktion in einem organischen aprotischen Lösungsmittel durchführt.

X¹ und X² in Formel II stehen bevorzugt entweder für -Cl oder -Br. Y steht bevorzugt für -Cl.

In einer anderen bevorzugten Ausführungsform stellen Y -Cl und X -Cl oder -Br dar.

Das Lösungsmittel ist bevorzugt ausgewählt aus der Gruppe der Kohlenwasserstoffe, Halogenkohlenwasserstoffe, Ether, Ketone, Carbonsäureester, Sulfone, N,N-Dialkylcarbonsäureamide, N-Alkyllactame und Lactone.

Einige Beispiele sind Petrolether, Pentan, Hexan, Cyclohexan, Methylcyclohexan, Benzol, Toluol, Xylol, Chlorbenzol, Methylenchlorid, Chloroform, 1,2-Dichlorethan, 1,1,2,2-Tetrachlorethan, Diethylether, Dibutylether, Ethylenglykoldimethylether, Tetrahydrofuran, Dioxan, Aceton, Methylisobutylketon, Essigsäuremethyl- oder -ethylester, Tetramethylensulfon, Dimethylformamid, N-Methylpyrrolidon, γ-Valerolacton und Butyrolacton.

Bevorzugt ist das Lösungsmittel polar und aprotisch. Ein besonders bevorzugtes Lösungsmittel ist Essigsäureethylester. Die Menge des Lösungsmittels kann in weiten Grenzen gewählt werden. Zweckmässig wird die gleiche bis zehnfache Menge Lösungsmittel bezogen auf die Menge der Verbindung der Formel II verwendet.

Halogenwasserstoffänger sind allgemein bekannt. Es kann sich z.B. um tertiäre Stickstoffbasen mit bevorzugt insgesamt 3 bis 20, besonders 3-12 C-Atomen handeln, um Alkalimetall- oder Erdalkalimetallsalze organischer Säuren oder Kohlensäure, oder um Alkalimetall- oder Erdalkalimetalloxide oder -hydroxide. Einige Beispiele sind Natriumcarbonat, Natrium- oder Calciumhydrogencarbonat, Natriumacetat, NaOH, KOH, MgO und CaO; oder aromatische, aliphatische oder cyclische tertiäre Stickstoffbasen wie z.B. Trimethyl-, Triethyl-, Tripropyl-, Tributyl-, Triethanol-oder Butyldimethylamin, Pyridin, 2,6-Dimethylpyridin, N-Methylpyrrolin und N-Methylmorpholin.

Bevorzugte Halogenwasserstoffänger sind tertiäre Stickstoffbasen und MgO. Besonders bevorzugt ist 2,6-Dimethylpyridin (Lutidin).

Der Halogenwasserstoffänger kann in geringem Unterschuss oder Ueberschuss, bezogen auf die Menge der Verbindung der Formel II, eingesetzt werden. Bevorzugt werden äquimolare Mengen verwendet.

Geeignete Edelmetallkatalysatoren sind z.B. Iridium, Rhodium, Platin, Ruthenium und Palladium. Besonders bevorzugt wird Palladium verwendet. Das Edelmetall wird bevorzugt als Trägerkatalysator eingesetzt. Beispiele für Träger sind BaSO₄, SiO₂, Al₂O₃ und insbesondere Aktivkohle. Der Träger kann 0,1 bis 20 Gew.-% des Edelmetalls enthalten. Es können auch sulfidierte Katalysatoren verwendet werden. Die eingesetzte Menge kann z.B. 0,1 bis 20 Gew.-% betragen, bezogen auf die Verbindungen der Formel II. Es kann zweckmässig sein, während der Reaktion neuen Katalysator zuzugeben.

Die Reaktionstemperatur beträgt bevorzugt 0 bis 80°C, besonders 0 bis 30°C. Der Druck beträgt bevorzugt bis zu 2 MPa. Bevorzugt wird die Reaktion bei Normaldruck durchgeführt.

Die Verbindungen der Formel II sind bekannt oder nach bekannten Verfahren durch α,α-Dichlorierung oder -bromierung von entsprechenden Butyraldehyden erhältlich. Solche Verfahren sind z.B. von P. Martin et al. in Tetrahedron 41, S. 4057 (1985) und H. Rimpler et al. in Chem. Ber. 118, S. 4288 (1985) beschrieben.

Die Verbindungen der Formel I sind wertvolle Zwischenprodukte zur Herstellung von in 5-Stellung substituierten 2-Acyl-1,3-cyclohexandionen, die als Herbizide und zur Regulierung des Pflanzenwachstums verwendet werden können (siehe EP-A-0 243 313). Die Verbindung der Formel I wird hierzu in an sich bekannter Weise zum Beispiel zum 1-Alkyl- oder -Benzylthio-1-cyclopropanaldehyd umgewandelt und in den in der EP-A- 0 243 313 beschriebenen Verfahren eingesetzt.

Die nachfolgenden Beispiele erläutern die Erfindung näher. Die Selektivität wird gaschromatographisch bestimmt.

### A) Herstellung von Ausgangsverbindungen

### Beispiel a: Herstellung von 2,2-Dibrom-4-chlorbutyraldehyd

110 g 4-Chlorbutyraldehyd, 286 g 5,5-Dibrombarbitursäure und 10 ml HBr in Essigsäure (33 %) werden in 600 ml Ether über Nacht gerührt. Das Reaktionsgemisch wird vom gebildeten Festkörper abfiltriert, 2x mit H₂O sowie 1x mit verdünnter NaHCO₃-Lösung gewaschen, getrocknet (Na₂SO₄), eingedampft und im Hochvakuum destilliert. Man erhält 81 g Produkt; Siedepunkt (Sdp.): 42-43°/1,3x 10⁻² mbar, Gehalt 90 % (gaschromatographische Bestimmung).

### B) Herstellungsbeispiele:

Beispiel 1: 351 g 2,2,4-Trichlorbutyraldehyd werden in 3 l Essigsäureethylester in Gegenwart von 214 g 2,6-Dimethylpyridin mit 18 g 5 % Pd/C-Katalysator bei Normaldruck und 5-22°C hydriert, bis die Wasserstoffaufnahme nach 7,5 Std. zum Stillstand kommt. Umsatz: 94,3 %, Selektivität: 99,9 %. Nach der Abtrennung der unlöslischen Anteile wird der Essigester abdestilliert. Die Vakuumdestillation des Rückstandes ergibt reinen 2,4-Dichlorbutyraldehyd in 84,5 %iger Ausbeute; Sdp.: 90°C (68 mbar).
Beispiel 2: 5,0 g 2,2,4-Trichlorbutyraldehyd werden in 50 ml Essigsäureethylester in Gegenwart von 3,05 g 2,6-Dimethylpyridin (2,6-Lutidin) mit 0,25 g 5 % Pd/C-Katalysator bei Normaldruck und 15-20°C hydriert. Die Wasserstoffaufnahme kommt nach 19 Std. zum Stillstand.
   Umsatz: 94,3 %, Selektivität: 99,9 %.
Beispiel 3: Wird wie in Beispiel 2 hydriert, aber an Stelle von 2,6-Lutidin 2,88 g Triethylamin verwendet, so beträgt die Hydrierdauer 19 Std..
   Umsatz: 86 %, Selektivität: 99,7 %.
Beispiel 4: Wird wie in Beispiel 2 verfahren, aber an Stelle von 2,6-Lutidin 1,15 g MgO verwendet, so beträgt die Hydrierdauer fast 7 Std., wobei die Hydrierung abgebrochen werden muss. Umsatz: 99,91 %, Selektivität: 96,5 %.
Beispiel 5: Wird wie in Beispiel 2 verfahren, aber an Stelle von 2,6-Lutidin 2,34 g Natriumacetat verwendet, so beträgt die Hydrierdauer 25 Std., wobei die Hydrierung abgebrochen werden muss.
   Umsatz: 65,7 %, Selektivität: 85,4 %.
Beispiel 6: Wird wie in Beispiel 2 hydriert, aber an Stelle von Essigsäureethylester Tetrahydrofuran verwendet, so resultiert eine Hydrierzeit von 18 Std., wobei während der Hydrierung frischer Katalysator zugesetzt und die Hydrierung bei der theoretischen Wasserstoffaufnahme abgebrochen werden muss.
   Umsatz: 96,05 %, Selektivität: 96,6 %.
Beispiel 7: Wird wie in Beisiel 2 hydriert, aber an Stelle von Essigsäureethylester Dioxan verwendet, so resultiert eine Hydrierzeit von 15 Stunden, wobei während der Hydrierung frischer Katalysator zugesetzt und die Hydrierung bei der theoretischen Wasserstoffaufnahme abgebrochen werden muss.
   Umsatz: 93,8 %, Selektivität: 96,6 %.
Beispiel 8: Wird wie in Beispiel 2 hydriert, aber an Stelle von Essigsäureethylester Methyl-t-butylether verwendet, so resultiert eine Hydrierzeit von 18 Std., wobei während der Hydrierung frischer Katalysator zugesetzt und die Hydrierung bei der theoretischen Wasserstoffaufnahme abgebrochen werden muss. Umsatz: 92,4 %, Selektivität: 93,7 %.
Beispiel 9: 5,0 g 2,2,4-Trichlorbutyraldehyd werden in 44 ml Essigsäureethylester in Gegenwart von 4,11 g Triethanolamin mit 0,3 g Raney-Nickel (mit Alkohol wasserfrei gewaschen) bei Normaldruck und 20-22°C hydriert. Es resultiert eine Hydrierzeit von 13 Std., wobei während der Hydrierung frischer Katalysator zugesetzt werden muss.
   Umsatz: 89 %, Selektivität: 94,4 %.
Beispiel 10: 5,0 g 2,2-Dibrom-4-chlorbutyraldehyd werden in 50 ml Essigsäureethylester in Gegenwart von 1,82 g 2,6 Lutidin mit 0,25 g 5 % Pd/C-Katalysator bei Normaldruck und 20-22°C hydriert. Während der Hydrierung muss frischer Katalysator zugegeben werden. Nach 3 Std. wird die Hydrierung abgebrochen.
   Umsatz: 100 %, Selektivität: 88 %.
Beispiel 11: 76 g 2,2-Dibrom-4-chlorbutyraldehyd werden analog Beispiel 10 hydriert. Nach 2 Std. wird die Hydrierung abgebrochen. Nach Abtrennung der unlöslichen Anteile und des Lösungsmittels erfolgt eine Destillation. Es resultieren 39,5 g 2-Brom-4-chlorbutyraldehyd (Gehalt: 95 %), Sdp.: 78-81°C/22 mbar, entsprechend einer Ausbeute von 70,4 % d.Th.

## Patentansprüche

1. Verfahren zur Herstellung von Dihalogenbutyraldehyden der Formel I 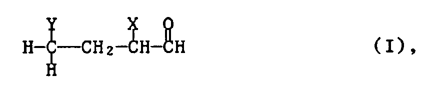 worin
X und Y unabhängig voneinander -Cl oder -Br darstellen, durch katalytische Dehalogenierung von α,α-Dihalogenverbindungen mit Wasserstoff in Gegenwart eines Edelmetallkatalysators oder Raney-Nickel, eines Halogenwasserstoffängers und eines Lösungsmittels bei einer Temperatur von 0-150°C und Normaldruck oder einem Druck bis zu 15 MPa, dadurch gekennzeichnet, dass man als α,α-Dihalogenverbindung einen α,α-Dihalogenbutyraldehyd der Formel II 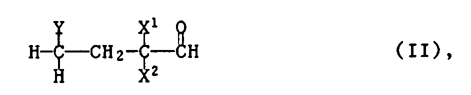 worin Y die zuvor angegebene Bedeutung hat und X¹ und X² unabhängig voneinander -Cl oder -Br darstellen, verwendet und die Reaktion in einem organischen aprotischen Lösungsmittel durchführt.

2. Verfahren gemäss Anspruch 1, dadurch gekennzeichnet, dass X¹ und X² entweder für -Cl oder -Br stehen.

3. Verfahren gemäss Anspruch 1, dadurch gekennzeichnet, dass Y -Cl ist und X -Cl oder -Br ist.

4. Verfahren gemäss Anspruch 1, dadurch gekennzeichnet, dass das Lösungsmittel ausgewählt ist aus der Gruppe der Kohlenwasserstoffe, Halogenkohlenwasserstoffe, Ether, Ketone, Carbonsäureester, Sulfone, N,N-Dialkylcarbonsäureamide, N-Alkyllactame und Lactone.

5. Verfahren gemäss Anspruch 1, dadurch gekennzeichnet, dass das Lösungsmittel polar und aprotisch ist.

6. Verfahren gemäss Anspruch 1, dadurch gekennzeichnet, dass das Lösungsmittel Essigsäureethylester ist.

7. Verfahren gemäss Anspruch 1, dadurch gekennzeichnet, dass es sich bei dem Halogenwasserstoffänger um eine tertiäre Stickstoffbase oder Magnesiumoxid handelt.

8. Verfahren gemäss Anspruch 7, dadurch gekennzeichnet, dass die Stickstoffbase 2,6-Dimethylpyridin ist.

9. Verfahren gemäss Anspruch 1, dadurch gekennzeichnet, dass es sich bei dem Edelmetallkatalysator um Palladium handelt.

10. Verfahren gemäss Anspruch 1, dadurch gekennzeichnet, dass es bei einer Temperatur von 0 bis 30°C und Normaldruck durchgeführt wird.

## Claims

1. A process for the preparation of a dihalobutyraldehyde of the formula I 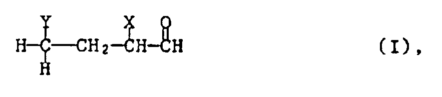 in which X and Y are each independently of the other Cl or Br, by catalytic dehalogenation of an α,α-dihalo compound with hydrogen in the presence of a noble metal catalyst or Raney nickel and of a hydrogen halide acceptor and a solvent, at a temperature of from 0-150°C and under normal pressure or under a pressure of up to 15 MPa, which process comprises using as α,α-dihalo compound an α,α-dihalobutyraldehyde of the formula II 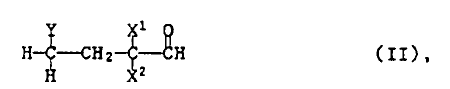 in which Y has the above meaning and X¹ and X² are each independently of the other Cl or Br, and carrying out the reaction in an organic aprotic solvent.

2. A process according to claim 1, wherein X¹ and X² are either Cl or Br.

3. A process according to claim 1, wherein Y is Cl and X is Cl or Br.

4. A process according to claim 1, wherein the solvent is selected from the group consisting of hydrocarbons, halogenated hydrocarbons, ethers, ketones, carboxylic esters, sulfones, N,N-dialkylcarboxamides, N-alkyl-lactams and lactones. the group consisting of hydrocarbons, halogenated hydrocarbons, ethers, ketones, carboxylic esters, sulfones, N,N-dialkylcarboxamides, N-alkyl-lactams and lactones.

5. A process according to claim 1, wherein the solvent is polar and aprotic.

6. A process according to claim 1, wherein the solvent is ethyl acetate.

7. A process according to claim 1, wherein the hydrogen halide acceptor is a tertiary nitrogen base or magnesium oxide.

8. A process according to claim 7, wherein the nitrogen base is 2,6-dimethylpyridine.

9. A process according to claim 1, wherein the noble metal catalyst is palladium.

10. A process according to claim 1, which is carried out at a temperature of from 0 to 30°C and under normal pressure.

## Revendications

1. Procédé pour préparer des dihalogéno-butyraldéhydes de formule 1 : 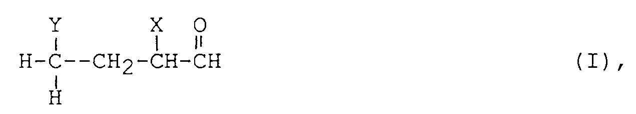 (dans laquelle :
X et Y représentent chacun, indépendamment l'un de l'autre, -Cl ou -Br), par déshalogénation catalytique de composés α,α-dihalogénés, avec de l'hydrogène en présence d'un catalyseur à base de métal noble ou de nickel de Raney, d'un accepteur d'un hydracide halogéné et d'un solvant à une température de 0 à 150°C et sous la pression normale ou une pression allant jusqu'à 15 Mpa, procédé caractérisé en ce qu'on utilise comme composé α,α-dihalogéné un α,α-dihalogéno-butyraldéhyde de formule II 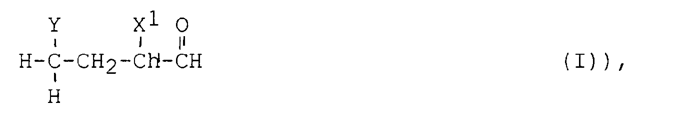 (dans laquelle Y a le sens indiqué ci-dessus, et X¹ et X² représentent chacun, indépendamment l'un de l'autre, -Cl ou -Br) et l'on conduit la réaction dans un solvant organique aprotique.

2. Procédé selon la revendication 1, caractérisé en ce que X¹ et x² représentent -Cl ou -Br.

3. Procédé selon la revendication 1, caractérisé en ce que Y représente -Cl et X représente -Cl ou -Br.

4. Procédé selon la revendication 1, caractérisé en ce que le solvant est choisi dans l'ensemble formé par les hydrocarbures, les hydrocarbures halogénés, les éthers, les cétones, les esters d'acides carboxyliques, les sulfones, les N,N-dialkylcarboxamides, les N-alkyllactames et les lactones.

5. Procédé selon la revendication 1, caractérisé en ce que le solvant est polaire et aprotique.

6. Procédé selon la revendication 1, caractérisé en ce que le solvant est l'acétate d'éthyle.

7. Procédé selon la revendication 1, caractérisé en ce qu'il s'agit, pour l'accepteur d'hydracide halogéné, d'une base azotée tertiaire ou de l'oxyde de magnésium.

8. Procédé selon la revendication 7 caractérisé en ce que la base azotée est la 2,6-diméthylpyridine.

9. Procédé selon la revendication 1, caractérisé en ce qu'il s'agit, pour le catalyseur à base de métal noble, de palladium.

10. Procédé selon la revendication 1, caractérisé en ce qu'il est conduit à une température de 0 à 30°C et sous la pression normale.
